# EUROPEAN PATENT APPLICATION

(11) **EP 2 286 784 A1**
(43) Date of publication of application: **23.02.2011**
(21) Application number: 10008004.3
(22) Date of filing: 30.07.2010
(51) Int. Cl.: A61K 6/083, A61L 24/12

(54) **Tooth-adhesive composition**

(30) Priority: 20.08.2009 JP 2009190937
(71) Applicant: GC Corporation, Itabashi-ku Tokyo 174-8585 (JP)
(72) Inventor: Yarimizu, Hideki, Tokyo 174-8585 (JP); Tokui, Hideki, Tokyo 174-8585 (JP); Nagao, Sakaya, Tokyo 174-8585 (JP)
(74) Representative: Müller-Boré & Partner Patentanwälte

(57) **Abstract**

To provide a tooth-adhesive composition not causing lowering of preservation stability due to a reaction between a filler having a X-ray imaging property and an acid component in the composition, and not having necessity for mixing and kneading of components separated in two or more packages unlike a conventional case, the tooth-adhesive composition includes (a) a (meth)acrylate compound having an acid group, (b) a filler which is an alumino-silicate glass powder including Sr and/or Ba and/or Ca, and (c) a photopolymerization catalyst, where the filler does not substantially react with the (meth)acrylate compound having an acid group, which is the a) component, by making Al₂O₃ content to be 15% or less by weight in terms of oxides, and these (a), (b), and (c) components co-exist in one pack.

## Description

The present invention relates to a tooth-adhesive composition used in tooth restorative treatments.

Dental composite resins have been widely used in tooth restorative treatments, where the dental composite resins include monomers, and/or oligomers, and/or prepolymers of methacrylate compounds and/or acrylate compounds which have radical polymerization property, and include inorganic fillers. However, since the dental composite resin itself does not have adhesive property with the tooth structure, it is indispensable for the dental composite resin to be used together with tooth adhesives which are called dental bonding materials including acid components. Since there may occur a big difference about an adhesive property of the dental bonding material according to a usage of a dentist, the bonding materials often become a cause of a variation in a restorative treatment.

On the other hand, as for conventional tooth adhesives, glass ionomer cements and adhesive resin cements have been used besides the bonding materials. These tooth adhesives are provided to dentists in a form of two or more packs including, for example, a powder and a liquid or a liquid (paste) and a liquid (paste). These components are mixed each other at a predetermined ratio by dentists just before use, and applied. However, if the dentists do not appropriately weigh or mix these components, variations in the restorative treatment could result.

The reason that the conventional tooth adhesive is provided in a form of two or more packs is for preventing ion reaction between a filler containing metals and an acid component. The filler containing metals gives X-ray imaging property which is required for the tooth adhesive as a dental material, and the acid component is indispensable for giving adhesive strength to the tooth adhesive. That is, when both components are filled in one pack, an ion reaction occurs between both components during storage of the tooth adhesive so that the components gel, and the ion reaction reduces the acid component necessary for the tooth adhesive so that the tooth adhesive strength could decrease. Therefore, in the conventional tooth adhesive, it is necessary to separately pack and store the acid component and the filler containing metals, and an operation for mixing both the components is required when using the tooth adhesive.

As for a conventional technique, Japanese Patent Application Laid-Open No. 62-149715 discloses a mixture combining a monomer having an acid group, a metal compound, and a curing agent. However, the mixture is produced assuming that these materials packed in plural packs are mixed at a time of use, so that the mixture cannot be prevented from causing variations due to kneading at a time of practical use. Further, Japanese Patent Application Laid-Open No. 62-149715 discloses a composition in one pack, in which calcium hydroxide and barium sulfate (both do not generate an ion reaction with an acid component but have X-ray imaging property) are used as a filler, in the exemplary embodiment 7. However, since a cured body becomes extremely opaque in this case, the composition cannot be used for a filler particularly. Furthermore, Japanese Patent Application Laid-Open No. 62-149715 discloses a composition in one pack, which includes polyacrylated polymaleic acid and a glass ionomer cement powder (having transparency but generating an ion reaction with an acid component) together, in the exemplary embodiment 12. However, the components are easy to gel due to acid-base reaction in the composition in this combination, so that the composition cannot have sufficient preservation stability in a practical use.

Furthermore, Japanese Translation of PCT Publication No. 2004-529946 discloses a self-adhesive dental material combining an ethylenic unsaturated compound having an acid group and a filler reacting with an acid component. However, since the composition includes the acid component and the reactive filler together in one pack, the components are easy to gel due to an ion reaction during storage of the material, so that it is impossible to acquire the sufficient preservation stability in a practical use.

The present invention is directed to a tooth-adhesive composition which does not cause lowering of preservation stability due to a reaction between a filler having an X-ray imaging property and an acid component for acquiring tooth adhesive strength, while the filler having the x-ray imaging property is used, the cured material has high transparency, and the material does not have necessity for mixing and kneading of components separated in two or more packs at a time of use, unlike conventional cases.

Present inventors carried out earnest works to solve the aforementioned problems, and as a result, they found out the followings to complete the present invention. When alumino-silicate glass powder having Al₂O₃ content of 15% or less by weight in terms of oxides, and including Sr and/or Ba and/or Ca is used as a filler component together with a (meth)acrylate compound having acid groups as acid components, the filler component does not generate ion reaction with the acid component while the filler component has sufficient X-ray imaging property as a dental material. Thus, the tooth-adhesive composition can have high preservation stability and can give transparency to the cured body.

According to an aspect of the present invention, a tooth-adhesive composition includes
(a) a (meth)acrylate compound having an acid group,
(b) a filler made from an alumino-silicate glass powder containing Sr and/or Ba and/or Ca, where the filler has an Al₂O₃ content of 15% or less by weight in terms of oxides, so that the filler does not substantially generate an ion reaction with the (meth)acrylate compound having an acid group as the (a) component, and (c) a photopolymerization catalyst,
   These (a), (b), and (c) components co-exist in one pack.

According to the tooth-adhesive composition of the present invention, while a filler having X-ray imaging property is used, the filler does not react with an acid component for acquiring tooth adhesive strength and does not decrease preservation stability. In addition, the tooth-adhesive composition has transparency, and does not have necessity for mixing and kneading of components separated in two or more packs, unlike conventional cases.

A (meth) acrylate compound having an acid group in the present invention is cured by a polymerization reaction, and comes to be a part of a base material of a composition. Simultaneously, the (meth)acrylate compound has effects for giving, to a tooth-adhesive composition, adhesive strength to the tooth structure, and ceramics such as zirconia and alumina, or alloys including noble metals, which are materials for a dental restoration. The (meth)acrylate compound in the present invention means various kinds of acrylate or methacrylate monomers, oligomers, and prepolymers. The (meth)acrylate compound having an acid group is preferably (meth)acrylate having a phosphate group or a carboxyl group as the acid group, and the (meth)acrylate having one or more phosphate groups or carboxyl groups in one molecule can be used. Since the phosphate groups have acidity stronger than that of the carboxylic group, the (meth)acrylate having the phosphate group has the high effects for dissolving a smear layer of a tooth surface and for decalcifying tooth. Particularly, the (meth)acrylate having the phosphate group exercises the effect of high adhesiveness to enamel.

The (meth)acrylate compound having a phosphate group could be 2-(meth)acryloyloxyethyldihydrogen phosphate, bis[2-(meth)acryloyloxyethyl]hydrogen phosphate, 2-(meth)acryloyloxyethylphenylhydrogen phosphate, 6-(meth)acryloyloxyhexyldihydrogen phosphate, 6-(meth)acryloyloxyhexylphenylhydrogen phosphate, 10-(meth)acryloyloxydecyldihydrogen phosphate, 1, 3-di(meth)acryloylpropane-2-dihydrogen phosphate, 1, 3-di(meth)acryloylpropane-2-phenylhydrogen phosphate, bis[5-{2-(meth)acryloyloxyethoxycarbonyl} heptyl] hydrogen phosphate, or the like. Particularly, 10-(meth)acryloyloxydecyldihydrogen phosphate is preferable because of having excellent adhesive property and self-stability of a (meth)acrylate compound. These (meth)acrylate compounds having the phosphate group can be used independently or by mixing two or more kinds.

The (meth)acrylate compound having the carboxyl group could be 4-(meth)acryloxyethyltrimellitic acid, 4-(meth)acryloxyethyltrimellitic acid anhydride, 4- (meth) acryloxydecyltrimellitic acid, 4- (meth) acryloxydecyltrimellitic acid anhydride, 11-(meth)acryloyloxy-1, 1-undecanedicarboxylic acid, 1, 4-di(meth)acryloyloxypyromellitic acid, 2-(meth)acryloyloxyethylmaleic acid, 2-(meth)acryloyloxyethylphthalic acid, 2-(meth)acryloyloxyethylhexahydrophthalic acid, or the like. Particularly, 4-(meth)acryloxyethyltrimellitic acid and 4-(meth)acryloxyethyltrimellitic acid anhydride are preferable because of having an excellent adhesive property.

The alumino-silicate glass powder is used as a main component of a powder component to give transparency and X-ray imaging property, and can secure a certain degree of transparency when the alumino-silicate glass power is used with the (meth)acrylate compound. The alumino-silicate glass powder is required to include Al³⁺, Si⁴⁺, and O²⁻ as main components, and further include Sr and/or Ba and/or Ca. Particularly, the alumino-silicate glass powder is required to have 15% or less by weight of the content of Al₂O₃ in terms of oxides with respect to the total weight of glass. When the content exceeds 15% by weight, the alumino-silicate glass powder comes to have high acid reactivity, so that the alumino-silicate glass powder cannot co-exist with the (meth) acrylate compound having an acid group in one component. More particularly, the content is preferably 10% or less by weight. In addition, the words "the filler does not substantially generate an ion reaction" in the present invention does not mean that a filler does not generate an ion reaction at all with an acid component, but means that a filler could generate a very slight ion reaction in a range that the ion reaction between the acid component and the filler does not gelate the tooth-adhesive composition, and the ionic reaction does not affect stability for the dental product, when the filler blends with an acid component in one composition.

The alumino-silicate glass powder used in this present invention can be surface-modified by alkoxysilane, such as γ-methacryloxypropyltrimethoxysilane, vinyltrichlorosilane, vinyltriethoxysilane, vinyltrimethoxysilane, vinyltriacetoxysilane, vinyltri (methoxyethoxy) silane, or the like. Further, the alumino-silicate glass powder can react with a phosphoric acid and/or a polymer of α-β unsaturated carboxylic acid before the alumino-silicate glass powder is surface-modified. In addition, content of Al₂O₃ in the glass is defined as a content at a time of recalculating the Al₂O₃ as oxides based on an element ratio measured by fluorescent X-ray analysis of the glass. Thus, the content of Al₂O₃ in the glass does not mean the amount of Al blended as oxides at a time of producing the glass.

A polymerization reaction used for the tooth-adhesive composition according to the present invention is a radical polymerization reaction generated by a photopolymerization catalyst. As the photopolymerization catalyst, the combination of a sensitizing material and a reducing material is used in general. The sensitizing material could be camphorquinone, benzyl, diacetyl, benzyl dimethyl ketal, benzyl diethyl ketal, benzyl di(2-methoxyethyl) ketal, 4,4'-dimethylbenzyl-dimethyl ketal, anthraquinone, 1-chloroanthraquinone, 2-chloroanthraquinone, 1,2-benzanthraquinone, 1-hydroxyanthraquinone, 1-methylanthraquinone, 2-ethylanthraquinone, 1-bromoanthraquinone, thioxanthone, 2-isopropylthioxanthone, 2-nitrothioxanthone, 2-methylthioxanthone, 2,4-dimethylthioxanthone, 2,4-diethylthioxanthone, 2,4-diisopropylthioxanthone, 2-chloro-7-trifluorothioxanthone, thioxanthone-10, 10-dioxide, thioxanthone-10-oxide, benzoin methyl ether, benzoin ethyl ether, isopropyl ether, benzoin isobutyl ether, benzophenone, bis(4-dimethylaminophenyl) ketone, 4,4'-bisdiethylaminobenzophenone, a compound including an azide group, or the like. The sensitizing material is used by mixing one or more kinds.

Tert-amine is used as a reducing agent in general. Preferably the tert-amine could be dimethylaminoethyl methacrylate, triethanolamine, methyl 4-dimethylaminobenzoate, ethyl 4-dimethylaminobenzoate, or isoamyl 4-dimethylaminobenzoate. In addition to those reducing agents, benzoyl peroxide, an organic metal compound, a sulfinic acid derivative, and the like, can be used as a reducing material. In such photopolymerization type dental restorative material composition, the polymerization reaction can be attained by irradiating active rays such as ultraviolet rays, visible rays, or the like.

The tooth-adhesive composition according to the present invention can include (meth) acrylate not having an acid group. More particularly, the (meth)acrylate compound not having an acid group used in the present invention could be methyl (meth)acrylate, ethyl (meth)acrylate, isopropyl (meth)acrylate, n-butyl (meth)acrylate, isobutyl (meth)acrylate, hydroxypropyl (meth)acrylate, tetrahydrofurfryl (meth)acrylate, glycidyl (meth)acrylate, 2-hydroxyethyl (meth)acrylate, 2-hydroxypropyl (meth)acrylate, 3-hydroxypropyl (meth)acrylate, 2-methoxyethyl (meth)acrylate, 2-ethoxyethyl (meth)acrylate, 2-methoxyethyl (meth)acrylate, 2-ethylhexyl (meth)acrylate, benzyl (meth)acrylate, 2-hydroxy-1, 3-di(meth)acryloxy propane, ethylene glycol di(meth)acrylate, diethylene glycol di(meth)acrylate, triethylene glycol di(meth)acrylate, butylene glycol di(meth)acrylate, neopentyl glycol di(meth)acrylate, 1, 3-butanediol di(meth)acrylate, 1, 4-butanediol di(meth)acrylate, 1, 6-hexanediol di(meth)acrylate, trimethylolpropane tri(meth)acrylate, trimethylolethane tri(meth)acrylate, pentaerythritol tri(meth)acrylate, trimethylolmethane tri(meth)acrylate, pentaerythritol tetra(meth)acrylate, polybutylene glycol di(meth)acrylate, or bisphenol A diglycidyl (meth)acrylate. A monomer, oligomer, and prepolymer of these compounds can be preferably used. Further, as for (meth)acrylate having urethane bond, di-2-(meth)acryloxyethyl-2, 2, 4-trimethylhexamethylene dicarbamate, 1, 3, 5-tris [1, 3-bis{(meth)acryloyloxy}-2-propoxycarbonylamino hexane]-1, 3, 5-(1H, 3H, 5H) triazine-2, 4, 6- trione, and 2, 2-bis-4-(3-(meth)acryloyloxy-2-hydroxypropyl)-phenylpropane, can be used. In addition, the (meth)acrylate having urethane bond could be (meth)acrylate of urethane oligomer including 2, 2'-di(4-hydroxycyclohexyl) propane, 2-oxypanone, hexamethylene diisocyanate, and 2-hydroxyethyl (meth)acrylate, and (meth)acrylate of urethane oligomer including 1, 3-butanediol, hexamethylene diisocyanate, and 2-hydroxyethyl (meth)acrylate. These can be used independently or by mixing two or more kinds.

The tooth-adhesive composition according to the present invention can include a filler component not reacting with the (meth)acrylate compound having an acid group, which is (a) component, in addition to the alumino-silicate glass powder. The filler component has an effect for adjusting fluidity of the tooth-adhesive composition. The filler could be powder of anhydrous silicic acid, glasses such as barium glass, alumina glass, potassium glass, aluminosilicate glass and the like, synthetic zeolite, calcium phosphate, feldspar, fumed silica, aluminum silicate, calcium silicate, magnesium carbonate, hydrous silicic acid, hydrous calcium silicate, hydrous aluminum silicate, quartz, or the like. In order to bond with (meth) acrylate, the filler can be subjected to a surface treatment with a silane coupling agent, like the alumino-silicate glass powder. Further, an organic/inorganic composite filler produced by previously mixing the filler with a monomer or an oligomer of the (meth)acrylate compound, curing the mixture, and thereafter pulverizing the cured body can be used. These fillers can be used independently or by mixing two or more kinds. In addition, an opaque glass such as a barium glass or the like can be blended in a range of not giving an adverse influence on transparency of the composition after curing.

The polymerization composition according to the present invention can properly include, of course a polymerization inhibitor, an antibacterial agent, a pigment, and the like, which are conventionally used according to necessity. Further, the polymerization composition can include water in a liquid component for increasing a reactivity of the (meth)acrylate having an acid group to the tooth structure.

### [Example]

A tooth-adhesive composition was produced with blending ratios (% by weight) illustrated in Table 3, and subjected to tests for adhesive property and tests for preservation stability and X-ray imaging property.

Brevity codes in Table 3 are as follows.
TEGDMA: Triethylene glycol dimethacrylate
UDMA: Di-2-methacryloxyethyl-2,2, 4-trimethylhexamethylene dicarbamate
Aerosil: Fumed silica (commercial name R812, produced by Nippon Aerosil Co., Ltd.)

Table 1 shows blending ratios at a time of producing the alumino-silicate glass powder. In Table 1, the alumino-silicate glass powders I, II and III are used for the present invention, and the alumino-silicate glass powder IV has high blending ratio of Al₂O₃.

**<Table 1> % by weight**

| | I | II | III | IV |
|---|---|---|---|---|
| SiO₂ | 55 | 50 | 55 | 44 |
| Al₂O₃ | 10 | 10 | 10 | 21 |
| BaO | | 30 | 25 | |
| SrO | 20 | | | |
| B₂O₃ | 15 | 10 | 10 | |
| CaF₂ | | | | 12 |
| Ca₃(PO₄)₂ | | | | 14 |
| SrCO₃ | | | | 9 |
| Total | 100 | 100 | 100 | 100 |

The alumino-silicate glass powder was produced by fully mixing raw materials, putting the mixture in a high temperature electric furnace at 120°C for 5 hours so as to fuse a glass, cooling the fused glass, pulverizing the glass for 10 hours using a ball mill, and sieving the pulverized glass with 200 meshes (ASTM).

The content of Al₂O₃ in terms of oxides is defined as the content at a time of recalculating Al₂O₃ as oxides based on an element ratio measured by fluorescent X-ray analysis of the glass. Regarding a measuring method in the present example, the glass powder is pelletized by a press machine to have a diameter of 31mm and a thickness of 5mm, and the pelletized glass powder is measured at a tube voltage of 50kV and a measurement range diameter of 20mm. Table 2 shows these results.

**<Table 2> % by weight**

| | I | II | III | IV (Comparative example) |
|---|---|---|---|---|
| Al₂O₃ | 8 | 8 | 7 | 19 |

### [Adhesion test]

In each example and comparative example, a bending test was carried out according to ISO/11405:2003 5.2.4. That is, an extracted tooth of bovine was ground from a lip side surface with a water-resistant grinding paper of #600, and an area of adhering surface was restricted to have a diameter of 3 mm by a plastic tape (having a thickness of 0.1mm). A composition was applied on an adhering surface, and was pressed by a slide glass through the plastic film. The composition was light cured for 20 seconds by a LED light irradiator (Product name G-Light, produced by GC Corporation). After removing the slide glass and the plastic film, a stainless rod having a diameter of 10mm was adhered to the surface of the cured composition with a dental glass ionomer cement (Product name FUJI LUTE, produced by GC Corporation). The cured composition was allowed to stand for 1 hour at 37°C and humidity of 100%, and then was soaked in distilled water at 37°C for 23 hours. After taking out the composition from the distilled water, a tensile test was performed at the cross head speed of 1 mm/min. It was confirmed that the composition has a sufficient adhesive property as a material for dental adhesion.

### [Test for preservation stability]

In each example and comparative example, the composition was filled in an injection syringe type vessel having an injection port diameter of about 0.5mm, and was held at 45°C. Then, extrudability from the vessel was confirmed. When the composition can be extruded easily from the vessel, such composition was marked to be "A". When the composition gelled due to an ion reaction and is not extruded at all, such composition was marked to be "B".

Clearly from the test for confirming preservation stability in Table 3, when the alumino-silicate glass powders having Al₂O₃ of 15% or less were used, the compositions had excellent preservation stability.

### [X-ray imaging property]

A test was carried out according to IS04049-2000, and the X-ray imaging property of the composition was evaluated as X-ray imaging property corresponding to a thickness (mm) of an aluminum plate. It was confirmed that the composition had sufficient X-ray imaging property required for a dental material.

**<Table 3>**

| | | Example 1 | Example 2 | Example 3 | Example 4 | Example 5 | Example 6 | Comparative example 1 | Comparative example 2 |
|---|---|---|---|---|---|---|---|---|---|
| (Meth)acrylate compound having an acid group | 10-(meth)acryloyl oxydecyldihydrogen phosphate | 30 | | | 10 | | | 30 | |
| | Bis[2-(meth)acryloyl oxyethyl]dihydrogen phosphate | | 30 | | | 10 | | | |
| | 2-(meth)acryloyl oxyethyldihydrogen phospohate | | | 30 | | | 10 | | 10 |
| Alumino-silicate glass powders | Alumino-silicate glass powder I | 64 | | | 64,8 | | | | |
| | Alumino-silicate glass powder II | | 64 | | | 64,8 | | | |
| | Alumino-silicate glass powder III | | | 64 | | | 64,8 | | |
| | Alumino-silicate glass powder IV | | | | | | | 64 | 64,8 |
| Photopolymerization catalyst | Camphorquinone | 0,3 | 0,3 | 0,3 | 0,3 | 0,3 | 0,3 | 0,3 | 0,3 |
| | 2,4,6-trimethylbenzoyl diphenylphosphineoxide | 0,8 | 0,8 | 0,8 | 0,4 | 0,4 | 0,4 | 0,8 | 0,4 |
| | Ethyl4-dimethylamino benzoate | 0,8 | 0,8 | 0,8 | 0,4 | 0,4 | 0,4 | 0,8 | 0,4 |
| (Meth)acrylate compound not having an acid group | UDMA | | | | 16 | 16 | 16 | | 16 |
| | TEGDMA | | | | 4 | 4 | 4 | | 4 |
| Other filler | Aerosil | 4 | 4 | 4 | 4 | 4 | 4 | 4 | 4 |
| Polymerization inhibitor | Butylhydroxytoluene | 0,1 | 0,1 | 0,1 | 0,1 | 0,1 | 0,1 | 0,1 | 0,1 |
| | Total | 100 | 100 | 100 | 100 | 100 | 100 | 100 | 100 |
| Adhesion test ISO/TS11405 | To bovine enamel (MPa) | 9,2 | 10 | 8,8 | 7,8 | 8,3 | 8,4 | 8,9 | 7,9 |
| Extrudability during storage at 45°C | To Bovine dentin (MPa) | 3,5 | 4,3 | 4,1 | 7,1 | 6,5 | 7,7 | 4,5 | 6,8 |
| | After production | ○ | ○ | ○ | ○ | ○ | ○ | ○ | ○ |
| | After one week | ○ | ○ | ○ | ○ | ○ | ○ | × | ○ |
| | After two weeks | ○ | ○ | ○ | ○ | ○ | ○ | × | × |
| | After 4 weeks | ○ | ○ | ○ | ○ | ○ | ○ | × | × |
| X-ray imaging property | Thickness of a corresponding Al plate (mm) | 2 | 2,5 | 2,5 | 2,1 | 2,5 | 2,5 | 2,5 | 2,5 |

## Claims

1. A tooth-adhesive composition comprising:
(a) a (meth)acrylate compound having an acid group
(b) a filler made from an alumino-silicate glass powder containing Sr and/or Ba and/or Ca, wherein the filler has an Al₂O₃ content of 15% or less by weight in terms of oxides, so that the filler does not substantially generate an ion reaction with the (meth) acrylate compound having an acid group as the (a) component; and
(c) a photopolymerization catalyst,
wherein these (a), (b), and (c) components co-exist in one pack.

2. The tooth-adhesive composition as claimed in claim 1,
wherein the content of Al₂O₃ in the component in terms of oxides is 10% or less by weight.

3. The tooth-adhesive composition as claimed in claim 1 or 2, further comprising:
a (meth)acrylate compound not having an acid group.

4. The tooth-adhesive composition as claimed in anyone of claims 1 to 3, further comprising:
a filler not reacting with the (a) component.
